(19) Europäisches Patentamt
European Patent Office
Office européen des brevets

(11) **EP 1 781 820 B1**

(12) **EUROPEAN PATENT SPECIFICATION**

(45) Date of publication and mention
of the grant of the patent:
**04.04.2018 Bulletin 2018/14**

(51) Int Cl.:
*C12Q 1/68* (2018.01)    *G01N 33/543* (2006.01)
*G01N 15/06* (2006.01)

(21) Application number: **05781014.5**

(22) Date of filing: **16.08.2005**

(86) International application number:
**PCT/IB2005/052702**

(87) International publication number:
**WO 2006/018811 (23.02.2006 Gazette 2006/08)**

(54) **A BIO-ELECTRONIC DEVICE**

EINE BIOELEKTRONISCHE VORRICHTUNG

UN DISPOSITIF BIOÉLECTRONIQUE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **20.08.2004 IE 20040559**

(43) Date of publication of application:
**09.05.2007 Bulletin 2007/19**

(73) Proprietor: **Magnomics, SA**
**3060-197 Cantanhede (PT)**

(72) Inventors:
• **GRAHAM, Daniel, Leonard, Vincent**
**Liverpool Merseyside L25 2SE (GB)**
• **TEIXEIRA DUARTE FERREIRA, Hugo, Alexandre**
**P-2795-049 Linda-a-Velha (PT)**
• **DA CONCEIÇÃO DIAS MARQUES FELICIANO,
Nuno, Filipe**
**Damaia P-2720-212 Amadora (PT)**
• **PEIXEIRO DE FREITAS, Paulo, Jorge**
**P-2785-732 Sao Domingos de Rana (PT)**
• **GALVIN, Paul, Timothy**
**Cork (PT)**

(74) Representative: **Atkins, James Gordon John**
**Kilburn & Strode LLP**
**Lacon London**
**84 Theobalds Road**
**London WC1X 8NL (GB)**

(56) References cited:
**WO-A-03/054523    US-A- 5 981 297**

• **LI GUANXIONG ET AL: "Detection of single
micron-sized magnetic bead and magnetic
nanoparticles using spin valve sensors for
biological applications" JOURNAL OF APPLIED
PHYSICS, AMERICAN INSTITUTE OF PHYSICS.
NEW YORK, US, vol. 93, no. 10, 15 May 2003
(2003-05-15), pages 7557-7559, XP012058215
ISSN: 0021-8979**
• **BASELT D R ET AL: "A biosensor based on
magnetoresistance technology" BIOSENSORS &
BIOELECTRONICS, ELSEVIER SCIENCE
PUBLISHERS, BARKING, GB, vol. 13, no. 7-8, 3
June 1998 (1998-06-03), pages 731-739,
XP002285269 ISSN: 0956-5663**
• **EDELSTEIN R L ET AL: "THE BARC BIOSENSOR
APPLIED TO THE DETECTION OF BIOLOGICAL
WARFARE AGENTS" BIOSENSORS &
BIOELECTRONICS, ELSEVIER SCIENCE
PUBLISHERS, BARKING, GB, vol. 14, no. 10/11,
January 2000 (2000-01), pages 805-813,
XP001069427 ISSN: 0956-5663**
• **LAGAE L ET AL: "On-chip manipulation and
magnetization assessment of magnetic bead
ensembles by integrated spin-valve sensors"
JOURNAL OF APPLIED PHYSICS, AMERICAN
INSTITUTE OF PHYSICS. NEW YORK, US, vol. 91,
no. 10, 15 May 2002 (2002-05-15), pages
7445-7447, XP012054843 ISSN: 0021-8979**
• **WIRIX-SPEETJENS R ET AL: "On-chip magnetic
particle transport by alternating magnetic field
gradients" IEEE TRANSACTIONS ON
MAGNETICS IEEE USA, vol. 40, no. 4, July 2004
(2004-07), pages 1944-1946, XP002358774 ISSN:
0018-9464**

- FERREIRA H A ET AL: "Biodetection using magnetically labeled biomolecules and arrays of spin valve sensors (invited)" JOURNAL OF APPLIED PHYSICS, AMERICAN INSTITUTE OF PHYSICS. NEW YORK, US, vol. 93, no. 10, 15 May 2003 (2003-05-15), pages 7281-7286, XP012058127 ISSN: 0021-8979

- GRAHAM D L ET AL: "Magnetoresistive-based biosensors and biochips" TRENDS IN BIOTECHNOLOGY, ELSEVIER PUBLICATIONS, CAMBRIDGE, GB, vol. 22, no. 9, 2 July 2004 (2004-07-02), pages 455-462, XP004552610 ISSN: 0167-7799

## Description

### Introduction

The invention relates to detection of biomolecules.

It is known to provide a magnetic field sensor to detect magnetically labelled biomolecules. However, present biosensor and biochip devices (based on different types of sensor) utilise either: conventional (slow, diffusion-controlled) liquid phase molecular recognition reaction conditions (such as nucleic acid hybridisation conditions in DNA microarrays), which are fixed for any one detection experiment; or they employ electric fields to enhance the rates of biomolecular immobilisation or hybridisation processes by charge effects, primarily to reduce assay times. These techniques offer little control over the molecular recognition (sensing) conditions, other than changing the pH, salt or chemical composition of the sample medium (usually an aqueous phase buffering medium) by fluid flow. This is also a relatively slow process.

The invention is therefore directed towards providing an improved bio-electronic device for detection of biomolecules.

US 5,981,297 describes a method and apparatus for detecting target molecules in a liquid phase. The apparatus monitors whether the target molecule has selectively bound to recognition agents on the surface of the magnetic field sensor by monitoring the output of the sensor.

Lagae et al, "On-chip manipulation and magnetisation assessment of magnetic bead ensembles by integrated spin-valve sensors", Journal of Applied Physics, volume 91, number 10, 2002 describes the manipulation and detection of magnetic beads on a semiconductor chip for biological applications. A spin-valve sensor detects the stray magnetic field that emanates from the ensemble of magnetic particles.

### Statements of Invention

According to the invention, there is provided a bioelectronic device comprising a current carrying conductor and an electromagnet enabling frequency modulated biomolecular interaction control via the application of varying magnetic fields to magnetically labelled biomolecules.

In one embodiment, further comprising a biomolecular sensor located in proximity to the conductor.

In another embodiment, the device is integrated in a chip.

In a further embodiment, the conductor is in a pattern having one or more lines in close proximity and the sensor is located between the lines.

In one embodiment, the sensor is a magnetoresistive sensor.

In another embodiment, the sensor and the conductor together form a microelectromagnetic unit arrayed onto the chip.

In a further embodiment, there is a plurality of said units on the chip, and they are electronically addressable.

In one embodiment, the chip surface is functionalised with a probe.

In another embodiment, the probe is patterned.

In a further embodiment, the micro-electromagnetic unit generates electromagnetic fields in two phases, a first DC phase to attract biomolecules, and a second phase with an alternating magnetic field and frequency modulation.

In one embodiment, the alternating magnetic field is in the plane of the chip and orthogonal to the direction of the proximal conductor.

In another embodiment, the DC and AC phases are applied simultaneously.

### Detailed Description of the Invention

The invention will be more clearly understood from the following description of some embodiments thereof, given by way of example only with reference to the accompanying drawings in which:

Fig. 1 is a representation of a current conductor of a bioelectronic device of the invention;
Fig. 2 is a representation of an alternative conductor, incorporating a spin valve sensor;
Fig. 3 is a representation of interlinking of sensing units;
Figs. 4 and 5 are diagrams showing operation of the device;
Fig. 6 is a diagram illustrating the device in use in perspective;
Fig. 7 includes cross-sectional and plan views of a device with relative size of a biomolecule illustrated;
Fig. 8 is a series of diagrams showing operation of the device in more detail;
Fig. 9 includes diagrams showing energy plots across the device;
Figs. 10 and 11 are diagrams of alternative devices of the invention; and
Figs. 12 and 13 are images showing a device active surface at different stages of use.

The invention provides a bio-electronic device of multiple microfabricated electronic units on a single chip. The device is used to controllably place, detect and manipulate biomolecules labelled with micrometer or nanometer sized magnetic beads or particles. Application domains include DNA chips, protein chips, biosensors, diagnostics and biomolecular interaction studies.

Magnetic fields either generated on-chip using current carrying structures or applied to the chip using an electromagnet effect the rapid focusing, manipulation and detection of magnetically labelled biomolecules and allow the study of a wide range of conditions on biomolecular interaction events including recognition events (e.g. DNA hybridisation) and biomolecular release (e.g. DNA dehybridisation).

The device comprises on-chip micro-fabricated current carrying metallic lines to facilitate the precisely controlled movement of magnetically labelled biomolecules at on-chip biosensor sites and the investigation of frequency modulated biomolecular interaction. A probe biomolecule (or species) is immobilised over a designated area of the chip within a defined current line structure fabricated with or without associated on-chip sensors (preferably magnetoresistive, magnetic-field, electric field or optical sensors) and magnetically labelled target biomolecules are introduced to the fluid on the chip (preferably using controlled fluid flow via microfluidic channels). The magnetically labelled target biomolecules (or species) are rapidly focussed at a specifically designed micron-sized electromagnetic unit consisting of the current carrying line and a sensor or array of sensors fabricated within the line, using a current passed through the line structure.

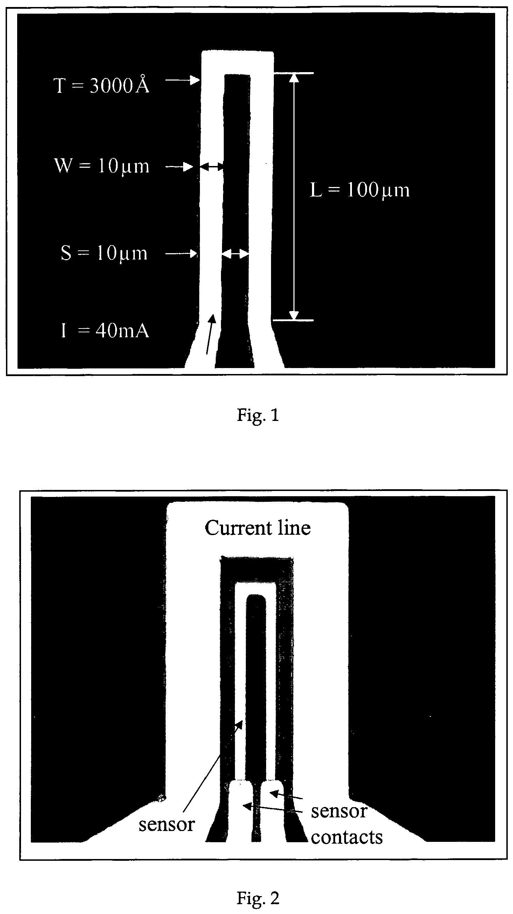

Referring to Fig. 1 a 'U-shaped' current line fabricated on-chip and passivated with silicon dioxide is shown. The dimensions of the line are represented by 't' (thickness), 'w' (width), 'l' (length), and 's'(space between the arms of the line). A function generator is used to apply an alternating magnetic field in the vicinity of the current line to enable the controlled movement of the magnetically labelled target species back and forth across the probe covered (sensor) area. Frequency modulation is then used to facilitate the manipulation of the interaction of the probe and target species. The magnetically labelled target biomolecules or species are repeatedly passed across the probe biomolecules or species within the unit at a speed dictated by the chosen frequency. The micro-electromagnetic unit can be used to control the contact time between the sensor bound biomolecule or species and the magnetically labelled biomolecule or species and to investigate the effect of different frequencies on the interaction of the two species e.g. biomolecular recognition events (such as nucleic acid hybridisation or protein-protein interaction) or biomolecular release mechanisms (such as the nucleic acid dehybridisation or the unbinding of proteins with particular affinity such as antibody-antigen). Biochip or biomolecular toolboxes based on the fabrication of numerous microfabricated electromagnetic units per chip potentially enable improved hybridisation efficiencies, diagnostic dehybridisation data, and highly specific discrimination between very similar target biomolecules such as single base mutations in nucleic acid sequences (single nucleotide polymorphisms SNPs), which are extremely important in the diagnosis of genetic diseases.

The device comprises on-chip microfabricated current (AC or DC) carrying metallic lines (e.g. composed of aluminium, copper or gold) with or without a magnetic component (e.g. nickel-iron cladding layer) and with variable design (geometry and geometrical dimensions), which facilitate, through the passage of current, the creation of current density gradients and consequently on-chip local magnetic fields of varying strength. The narrower parts of the fabricated lines, which produce a higher magnetic field are used to attract magnetic microspheres or nanoparticles (carriers, functionalised with biomolecules), to a particular region of the chip and also allow the precise manipulation of the movement of the magnetic labels in the immediate vicinity. A simple example of such a line design is shown in Fig.1.

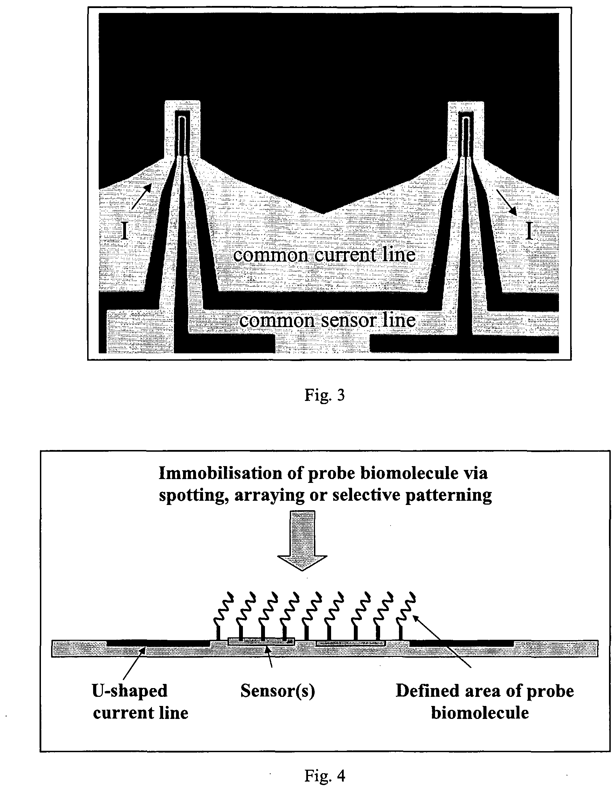

The device also includes a sensor or sensor array (magnetoresistive, magnetic or non-magnetic) integrated within the current carrying line design (in the area in which the magnetic labels are focussed and manipulated). An example shown in Fig.2 features a U-shaped magnetoresistive spin valve sensor fabricated within the U-shaped current line. The sensor or sensors could be linear, rectangular, square, U-shaped, meander or spiral in shape and may be a spin-valve, planar Hall sensor, magnetic tunnel junction, other magnetic field sensor or non-magnetic (e.g. optical) sensor in the case where the magnetic labels have an additional label enabling detection (e.g. fluorescence). The combination of the current carrying line and the sensor/s constitutes a 'micro-electromagnetic unit', which can be operated as a single unit, paired (Fig. 3) or arrayed on the chip (see 'prototype'). These units can be electronically addressed either individually or as pairs or groups simultaneously or sequentially. More specifically, Fig. 3 shows an example of how sensing units (current line and sensor/s) can be linked together using common current and sensor lines, facilitating a larger number of sensing units per chip. The current line is narrower at the U-shaped feature in order to increase the current density in that area of the line and hence increase the magnetic field produced in that area.

A 'probe' biomolecule or species of interest (biological, clinical, diagnostic or academic) to the user, which may be naturally occurring, biologically or chemically synthesized, chosen for the detection of the binding of a secondary, complementary target biomolecule is immobilised across the surface of the chip, spotted, arrayed or otherwise patterned preferably across the area of the sensor or sensing unit to achieve a single or multi-probe chip (Fig. 4). The immobilised biomolecule may be nucleic acid (DNA, RNA), a protein (antibody, receptor), a peptide, a synthetic molecule (synthon), a cell or a microorganism. Fig. 4 shows a cross-sectional scheme through the chip showing the preferred placement of probe biomolecules across the chip surface in the sensor or sensor-free zone between the two arms of the U-shaped current line.

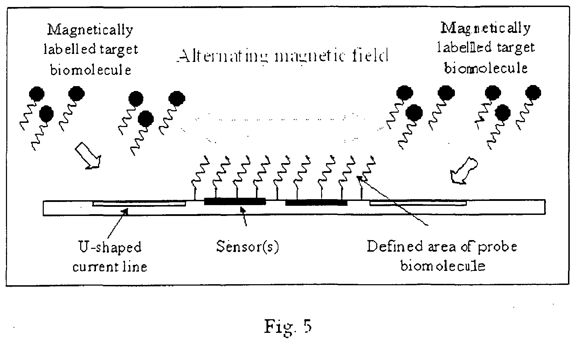

A magnetic label (or carrier) is functionalised with a 'target' biomolecule or species and introduced (using a pipette, a syringe or controlled microfluidics) into the fluid (usually a biological buffering medium) on the chip after positioning of the probe biomolecule/s or species (Fig. 5).

Fig. 5 shows a cross sectional scheme through the chip showing the attraction of magnetically labelled target biomolecules to the two arms of the U-shaped current line and the subsequent controlled movement of the magnetically labelled target biomolecules back and forth across the sensor zone between the two arms of the current line. The former is effected using a DC current through the U-shaped line and the latter via the application of an AC field using an electromagnet positioned above the chip. The magnetic label can vary in size (most probably from a few nanometers to a few micrometers in diameter), chemical and magnetic composition (polymer, copolymer matrix or encapsulated iron-oxide or transition metal ion containing) and magnetic content (5-100%). The magnetically labelled target species may be a biomolecule (a synthon, nucleic acid or protein) or (in principle) it may be a cell or microorganism.

An electromagnet, either external to the chip/chip carrier and positioned in such a way as to apply and regulate a magnetic field in the appropriate plane and direction on-chip (e.g. horseshoe magnet or coil), or an on-chip fabricated micro-electromagnet for the same use.

## Device Operation

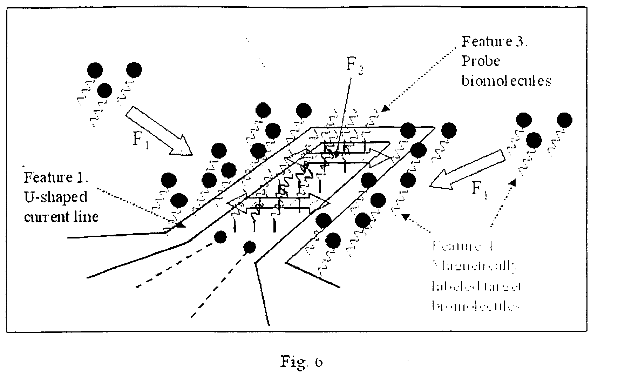

The functioning of a single micro-electromagnetic unit is represented schematically in Figs. 5 and 6. One method of using the device is as follows:

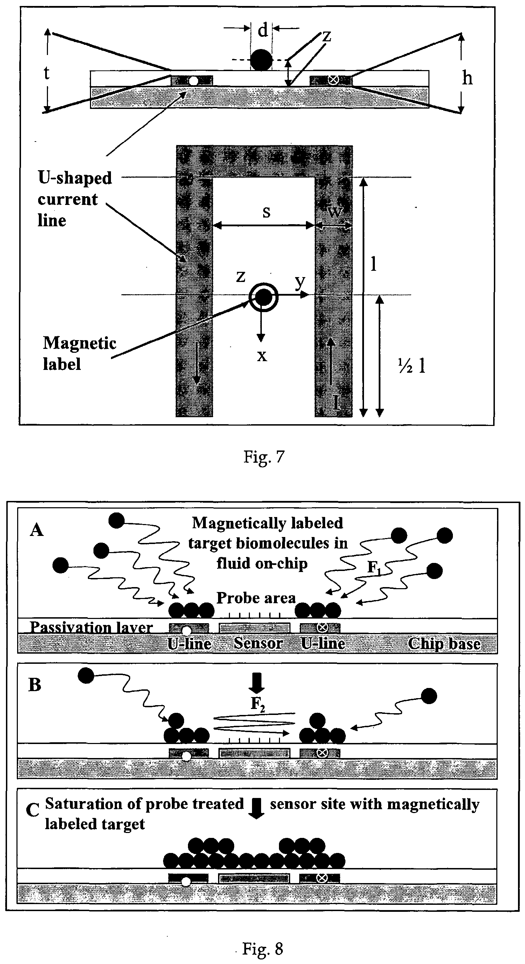

The chip surface is pre-functionalised with a particular probe or number of probe species, preferably patterned in the designated area or areas associated with the on-chip current line feature/s (e.g. within the arms of a U-shaped current line). The magnetically labelled target species or heterogeneous mixture (soup) of magnetically labelled target species is then introduced into the fluid (water or buffer) over the chip (Figs. 5/6). A current is then passed through the current line structure (Fig. 7) attracting and focussing the magnetically labelled target species at a particular region of the current line structure (e.g. both sides or arms of the U-shaped current line). When sufficient numbers of magnetically labelled targets have accumulated (usually on a timescale of seconds), an alternating magnetic field is then applied across the current line structure (e.g. across the U-shaped current line) in the plane of the chip and at a right angle to the length of U-shaped current line (see central arrow in Fig. 6).

In more detail, Fig. 6 is a schematic representation of a single functional unit of the invention showing the four basic features: (1) A 'U-shaped' current line; (2) A sensor or sensor-free area between the arms of the U-shaped current line (depicted here with a U-shaped spin valve sensor as in Fig.2); (3) Probe biomolecules immobilised over the sensor or sensor-free area and (4) Magnetically labelled target biomolecules introduced into the fluid on the chip. The U-shaped current line is used to create an attractive magnetic force (F1) using a DC current, focussing the magnetically labelled target biomolecules at each arm of the current line. An alternating magnetic field is then applied across the line structure to create a force (F2), which controllably moves the magnetically labelled target biomolecules back and forth from one arm of the U-shaped current line to the other across the probe biomolecule area. Fig. 7 shows a cross sectional (top) and top view (below) schematics of U-shaped current line structure showing the dimensions used to calculate the magnetic force or energy produced by the line on the magnetic label at a given current (I) and at a given distance from the current line. The current creates a horizontal (y-axis) magnetic field

$$\vec{H}_y = H_y \, \vec{e}_y$$

and a vertical magnetic field

$$\vec{H}_z = H_z \, \vec{e}_z$$

at a height

$$a = h + t + \frac{d}{2}$$

, t is the thickness of the passivating layer on the chip and h is the thickness of the current line and d is the diameter of the magnetic label.

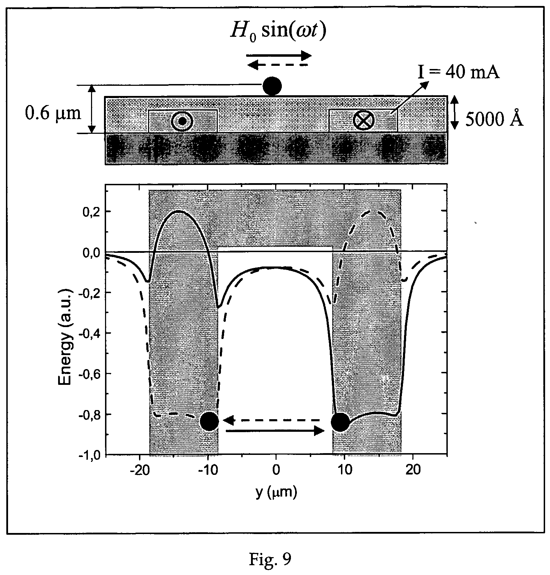

Referring to Fig. 8, this shows a cross sectional schematic to show: (a) How magnetically labelled target species are attracted to the arms of the U-shaped current line by a magnetic force $F_1$; (b) The alternating force $F_2$ that moves the magnetic labels back and forth across the probe functionalised area (within the U-shaped current line) as a result of the applied alternating magnetic field (magnetic label velocity being frequency dependent) and; (c) The way in which this process saturates the probe species area with magnetically labelled target species. Fig. 9 shows a cross sectional schematic through (top) and across (below) a U-shaped current line showing the direction of a current (I) through the line and an applied alternating magnetic field (H), producing a fluctuating magnetic energy profile (see chart, au = arbitrary units) effecting the movement of the magnetic labels from one arm of the U-shaped line to the other.

The alternating field causes the magnetically labelled targets to move back and forth from one side of the U-shaped structure to the other at a frequency dependent velocity (Fig. 8). During this period of operation the frequency may be varied as required effecting changes in the movement and behaviour of the magnetic labels, local temperature changes and potentially, chemical or conformational changes in the probe or target species. A profile showing the changes in magnetic energy which occur during the application of the alternating field is shown in Fig. 9.

## Alternative methods of use include :

Firstly, the DC current through the current line structure and the AC magnetic field may be applied simultaneously from the beginning of the experiment. Secondly, the nature of the current passed through the line may also be AC used in combination with a DC applied field (with or without an AC component). Thirdly, the current passed through the current line may have an AC and DC component in combination with an applied field having both AC and DC components. The applied field may also be in a vertical direction to the line as opposed to horizontal for other line geometries.

## This technique enables the experimentalist to :

(i) Focus and hence concentrate target species at sensor or sensor-free sites on-chip, enabling the use of low target concentrations.
(ii) To control the contact time (via magnetic label velocity) between probe and target species in order to optimise the binding of probe and target
(iii) To effect local temperature changes
(iv) To effect frequency dependent probe and target binding studies
(v) To effect frequency dependent probe and target release studies

## Structural Parameters

The current carrying metallic lines used to attract and manipulate the magnetically labelled biomolecules may be simple or complex in design. They may be single narrow lines, U-shaped, V-shaped (tapered), meander, spiral or circular (loop) used in combination with vertical or horizontal applied magnetic fields. The dimensions are on the micrometer scale, but could be as large as millimetre or as small as nanometer (which in principle could be used to attract and manipulate single nanometer-sized magnetic labels). As an example, the U-shaped current carrying metallic structures (Fig. 1) could be fabricated with dimensions of length (1) from $10\mu m$ to 1mm, width (w) $2\text{-}20\mu m$, space between the arms of the U-shape (s) $2\text{-}20\mu m$ and thickness (t) 100-500nm. However, functional structures with dimensions outside of these values are feasible.

The nature of the sensor (optional), probe species, target species and electromagnet may vary. The magnetic labels used may also vary in content, but would almost certainly be of nanometer to low micrometer in diameter.

## Operational Parameters

The current passed through the metallic structures (U-shaped or otherwise) has certain constraints. The minimum current must generate a magnetic field (in a certain region of the line e.g. the U-shape) of sufficient magnitude to attract and move magnetic labels of a particular mass, diameter and magnetic composition. The maximum current must not generate sufficient heat to damage the structure (or generate electromigration) and/or the probe and target biological species (denaturation or cell damage). For U-shaped structures with specific dimensions defined under 'structural parameters' above, the current passed may be >10 and <200mA. The alternating magnetic field applied to the structure via an on-chip or external electromagnet can also vary. The range of the alternating field that can be used depends on the device used to create the field (horseshoe magnet, coil or on-chip structure) and the current passed through the device. It is known that an increase in the field increases the force acting on the magnetic labels, but as yet the constraints on the magnitude of the field that can be used are unknown (i.e. the field used must be >0 Oe).

The range of the frequency applied to the magnetic labels in the desired region of the chip (preferably a sensor region) can be divided into two regimes: At a particular range (dependent on several features of the invention, including the current line structure and the magnetic labels used) the applied frequency effects the velocity with which the magnetic labels are moved back and forth across the desired region. For the current line structure featured in Fig. 1, a frequency range of 0.1-20Hz moves 250nm sized magnetic labels (Nanomag-D, Micromod, Germany) back and forth from one arm of the U-shaped line to the other at velocities which can easily be distinguished by eye via a microscope positioned above the chip. At frequencies above this range (for the stated current line structure) the magnetic labels no longer appear to move, but may vibrate. Thus once positioned in the desired region the interaction of a magnetically labelled target species with a probe species (preferably sensor-bound) can be studied at a much wider range of frequency (0.1Hz to GHz range). This frequency range can be used to perform biological interaction studies. The application of AC magnetic fields may also be used to effect on-chip local temperature changes (increase) by power absorption from the magnetic label.

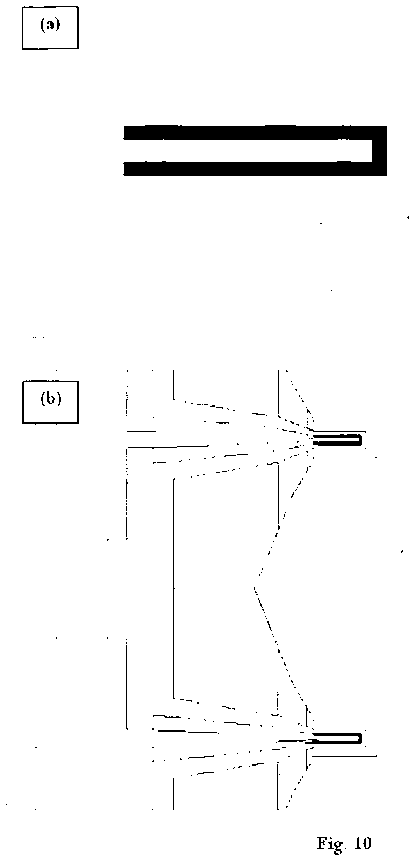

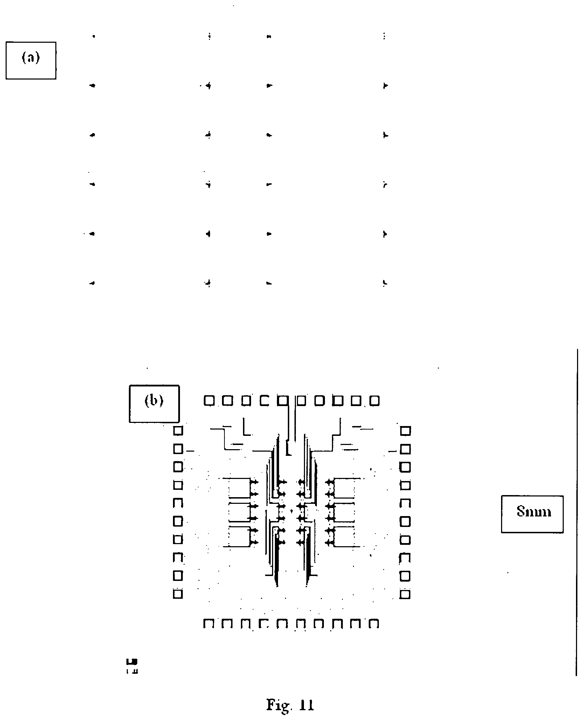

The micro-electromagnetic units described can be fabricated as single units or arrays on-chip to facilitate multi-probe or multi-analyte detection devices or biomolecular toolboxes for the study of biomolecular recognition. The first aspect of prototype design is the layout of the chip. The chip dimensions (usually mm scale) are defined and the available chip surface is used as efficiently as possible in such a way as to maximize the active sensing area within each sensing unit, to incorporate appropriate reference sensors and yet avoid electrical, magnetic or thermal cross-talk between sensors or on-chip current line structures. A differential sensor set-up uses a reference sensor in a Wheatstone bridge architecture to enable thermal and electrical drift compensation between a biologically active sensor and an biologically inactive sensor. The design of a first prototype based on the combination of U-shaped current line structures with spin-valves sensors is shown in Figs. 10 and 11. Fig. 10 shows a single micro-electromagnetic unit (a) of the device featuring a U-shaped current line structure (shaded) combined with a U-shaped magnetoresistive spin-valve sensor (black). These units can be fabricated and electronically addressed as individual units or connected to further units as shown in (b) two micro-electromagnetic units connected by common current and sensor lines. Note how the current line structure is tapered in width toward the U-structure in order to increase current density in the U-shaped parts of the line. Fig. 11 shows (a) an array of micro-electromagnetic units defined in the centre of a chip in four rows of six units, each comprised of one U-shaped current line and U-shaped spin valve sensor, (b) An 8 x 8mm chip showing the contact pads (□) and lines connecting current lines for the 4 x 6 array of micro-electromagnetic units. The connection lines are designed to allow units to be electronically addressed either individually, as pairs or as an array.

This chip was fabricated with twenty four U-shaped current line structures each having a U-shaped spin valve sensor fabricated within the arms of the U-shaped current line. A single micro-electromagnetic unit and a pair of such units fabricated with common connecting lines are shown in Fig. 10. An array of twenty four (four rows of six units) and the full chip layout (including contact pads) is shown in Fig. 11. The chips were fabricated on 3' silicon wafers using microelectronic processing techniques under cleanroom conditions. The structures are defined using laser lithography and sensor materials were deposited by sputtering. The integrity of the on-chip structures was then assessed via microscopic inspection and electrical resistance measurements. A protective (passivation) layer was deposited over the chip structures to prevent corrosion of the chip surface by fluids applied during the chemical and biological reactions. This material (substrate) should also provide ease of surface functionality for the immobilisation of biomolecules or cells and ideally show low nonspecific adherence of magnetic labels, polymers, proteins, nucleic acids and cells. Sputtered silicon dioxide or silicon nitride are suitable. Only the electrical contacts at the outer edges of the chip remain free of the passivation material. The wafer is then diced into individual chips (in this instance 8 x 8mm in dimension) and each chip is mounted in a chip carrier for easy connection to hardware. The contacts are wire bonded to the contact pads (shown as squares around the edge of the chip layout in Fig. 11b), which are then protected by a silicon gel or microfluidic channels are used to control fluid flow across the chip.

## Examples

A sensor-free chip was fabricated with U-shaped current lines to demonstrate the 'proof of principle' of the device using DNA-DNA hybridisation (i.e. the binding of a DNA probe with a complementary magnetically labelled DNA target) as a model for the use of the invention to (i) perform a rapid DNA hybridisation experiment and (ii) effect a frequency manipulated biomolecular recognition process.

## Chip and probe preparation

The on-chip U-shaped current line structures were fabricated with dimensions (Fig. 1) 100$\mu$m long, 10$\mu$m wide and 3000Å thick with a space between the arms of the U-shaped line of 10$\mu$m and passivated (covered) with a layer of silicon dioxide of 2000Å. Probe DNA molecules (50mer oligonucleotides corresponding to a specific sequence in the cystic fibrosis transmembrane regulator (CFTR) gene, where the most common cystic fibrosis mutation delF508 occurs) were immobilised across the full chip surface. The chip was placed within the electronic set-up (associated hardware) equilibrated in a hybridisation buffer (50mM histidine buffer) and positioned beneath the zoom lens of a microscope (Leica DMLM) fitted with a camera (JVC) in order to record images of the movement and binding (hybridisation) of the magnetic labels on the chip surface.

## Target preparation

Double stranded target DNA (consisting of a 96 bp sequence having a region of DNA complementary to the probe DNA sequence) was 3'-end biotinylated (Pierce kit) and incubated for 2 hours with 250nm Nanomag-D magnetic labels (Micromod) functionalised with streptavidin. This solution was then centrifuged in a benchtop centrifuge in order to spin down (pellet) the magnetic labels, which were then resuspended and washed in phosphate buffer, 100mM, pH7.2. This provided magnetic nanoparticles functionalised with probe-complementary target DNA. This preparation was boiled at 95°C for 5mins and immersed in ice prior to the experiment to produce single stranded magnetically labelled target DNA.

## Experimental Conditions

A current of 40mA (DC) was passed through the U-shaped current line structure and the magnetically labelled target DNA was added to the chip (to give a biological target concentration of approx. 100pM (picomolar). The magnetic labels were observed to move toward the U-shaped current line and accumulate on the top of the line. After 2 mins an alternating external magnetic field of +/-18.4 Oe rms was applied across the U-shaped line (at a right angle to the length of the arms of the U-shaped line in the plane of the chip). Simultaneously a pulse generator was used to apply a particular frequency to the chip. After 5 mins the process was stopped, the chip surface was washed with buffer (100mM phosphate buffer containing 150mM sodium chloride, pH 7.2) and an image of the chip surface in the region of the U-shaped line was recorded. The chip surface was then re-equilibrated in hybridiation buffer (50mM histidine) and the same process was then repeated for a period of 20mins and a further image of the chip surface was recorded. In one experiment, a frequency of 0.2Hz was used during the process and in a second identical experiment a frequency of 10Hz was applied.

**Results**

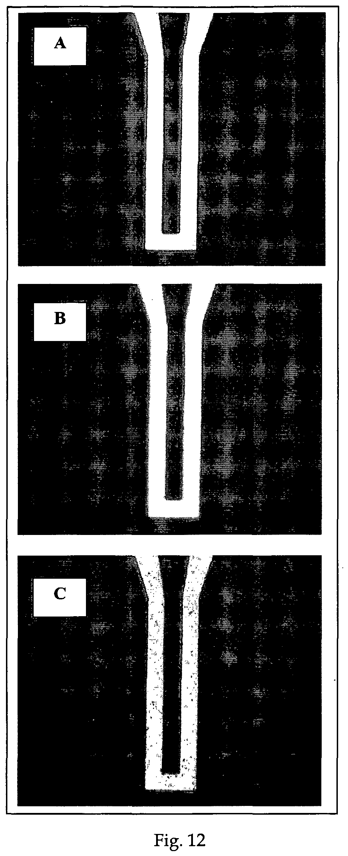

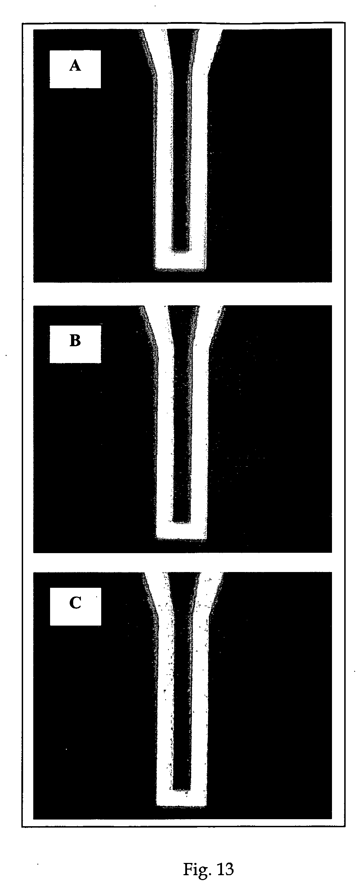

The respective images of the chip surface at times 0, after 5 mins and after a further 20mins are shown in Figs. 12/13 respectively, illustrating: (i) Rapid hybridisation (5-25mins) of probe and magnetically labelled target DNA; (ii) Increased hybridisation of the magnetically labelled target DNA with surface immobilised probe DNA in the area in between the arms of the U-shaped line as compared with the chip surface outside of the U-shaped line region and; (iii) A potential frequency effect on the degree of the hybridisation between the magnetically labelled target and the surface-bound probe.

In more detail, Fig. 12 shows images of an on-chip U-shaped current line structure at different stages during a magnetic field assisted DNA hybridisation experiment. A cystic fibrosis (CF) oligonucleotide probe (50mer) was immobilised across the whole chip surface and 250nm magnetic particles (Nanomag-D, Micromod, Germany) functionalised with the complementary target DNA sequence were introduced. A direct current of 40mA was passed through the line to attract the magnetic particles to the line and an alternating magnetic field of +/- 18.4 Oe rms and an applied frequency of 0.2 Hz. Hybridisation of the DNA was assessed by visualisation of the particles on and in between the line structure at time (a) 0 mins, and after washing at time (b) 5 mins and (c) 20mins operation of the device. Fig. 13 shows images of an on-chip U-shaped current line structure at different stages during a magnetic field assisted DNA hybridisation experiment. A cystic fibrosis (CF) oligonucleotide probe (50mer) was immobilised across the whole chip surface and 250nm magnetic particles (Nanomag-D, Micromod, Germany) functionalised with the complementary target DNA sequence were introduced. A direct current of 40mA was passed through the line to attract the magnetic particles to the line and an alternating magnetic field of +/- 18.4 Oe rms and an applied frequency of 10 Hz. Hybridisation of the DNA was assessed by visualisation of the particles on and in between the line structure at time (a) 0 mins, and after washing at time (b) 5 mins and (c) 20 mins operation of the device.

It will be appreciated that the invention offers both reduced assay times and the ability of the user to vary the biomolecular recognition conditions rapidly during the detection (sensing) process. The ability to change the way in which the molecular recognition process occurs in-situ, allows the user the potential to optimise the molecular interaction process and the detection process and to study the recognition process in an entirely new way. The invention allows the alteration of temperature and the determination of local temperatures on-chip, the control of contact time between sensor-bound probe biomolecules and (magnetic-field) controlled magnetically labelled target biomolecules and most significantly the ability to study biomolecular recognition processes at different applied frequencies. This offers the development of devices that can be used to perform unique frequency controlled on-chip biomolecular interaction experiments.

The invention would be attractive to licensees as it offers a biomolecular toolbox for the study of specific biological recognition processes and a detection system with the potential to discern small structural differences in heterogeneous biomolecular target compositions. This could be applied to drug screening, cell screening, antibody-antigen engineering or single base nucleic acid base mismatch mutation detection in the diagnosis of genetic diseases.

The invention is not limited to the embodiments described but may be varied in construction and detail.

**Claims**

1. A bioelectronic device integrated in a chip comprising:

   a current carrying conductor;
   a biomolecular sensor located in proximity to the conductor, wherein the conductor is in a pattern having a plurality of lines in close proximity and the sensor is located between the lines; and
   an electromagnet enabling frequency modulated biomolecular interaction control via the application of varying magnetic fields to magnetically labelled biomolecules,
   wherein the device is configured to generate electromagnetic fields in two phases, a first DC phase to attract the magnetically labelled biomolecules, and a second phase with an alternating magnetic field and frequency modulation to controllably move the magnetically labelled molecules back and forth from one line to another across the sensor, wherein the first and second phases are applied simultaneously.

2. A bioelectronic device as claimed in any of claim 1, wherein the sensor is a magnetoresistive sensor.

3. A bioelectronic device as claimed in claim 1 or 2, wherein the sensor and the conductor together form a micro-electromagnetic unit arrayed onto the chip.

**4.** A bioelectronic device as claimed in claim 3, wherein there is a plurality of said units on the chip, and they are electronically addressable.

**5.** A bioelectronic device as claimed in any preceding claim, wherein the chip surface is functionalised with a probe.

**6.** A bioelectronic device as claimed in claim 5, wherein the probe is patterned.

**7.** A bioelectronic device as claimed in any preceding claim, wherein the alternating magnetic field is in the plane of the chip and orthogonal to the direction of the proximal conductor.

**8.** A method of using the bioelectronics device of any preceding claim, comprising:

introducing magnetically labelled target species into a fluid over the chip;
passing a current through the current carrying conductor to generate the DC phase electromagnetic field to attract the magnetically labelled biomolecules; and
generating the second phase, alternating magnetic field to controllably move the magnetically labelled molecules back and forth from one line to another across the sensor, wherein the first and second phases are applied simultaneously.

**Patentansprüche**

**1.** Bioelektronische Vorrichtung, die in einem Chip integriert ist, Folgendes umfassend:

einen Strom führenden Leiter;
einen Biomolekularsensor, der in räumlicher Nähe zu dem Leiter angeordnet ist, wobei der Leiter eine Struktur mit mehreren Leitungen in enger räumlicher Nähe aufweist und der Sensor zwischen den Leitungen lokalisiert ist; und
einen Elektromagneten, der eine frequenzmodulierte biomolekulare Interaktionssteuerung über das Anlegen variierender Magnetfelder auf magnetisch markierte Biomoleküle ermöglicht, wobei die Vorrichtung konfiguriert ist, elektromagnetische Felder in zwei Phasen zu erzeugen, einer ersten Gleichstromphase, um die magnetisch markierten Biomoleküle anzuziehen, und einer zweiten Phase mit einem alternierenden Magnetfeld und einer Frequenzmodulation, um die magnetisch markierten Moleküle von einer Leitung zu einer anderen über den Sensor hinweg steuerbar vor und zurück zu bewegen, wobei die erste und die zweite Phase gleichzeitig angelegt werden.

**2.** Bioelektronische Vorrichtung nach Anspruch 1, wobei der Sensor ein Magnetowiderstandssensor ist.

**3.** Bioelektronische Vorrichtung nach Anspruch 1 oder 2, wobei der Sensor und der Leiter zusammen eine mikroelektromagnetische Einheit bilden, die auf dem Chip angeordnet ist.

**4.** Bioelektronische Vorrichtung nach Anspruch 3, wobei es mehrere der Einheiten auf dem Chip gibt und diese elektronisch adressierbar sind.

**5.** Bioelektronische Vorrichtung nach einem der vorhergehenden Ansprüche, wobei die Chip-Oberfläche mit einer Sonde funktionalisiert ist.

**6.** Bioelektronische Vorrichtung nach Anspruch 5, wobei die Sonde strukturiert ist.

**7.** Bioelektronische Vorrichtung nach einem der vorhergehenden Ansprüche, wobei das alternierende Magnetfeld in der Ebene des Chips und orthogonal zu der Richtung des proximalen Leiters ist.

**8.** Verfahren zum Verwenden der bioelektronischen Vorrichtung nach einem der vorhergehenden Ansprüche, Folgendes umfassend:

Einführen einer magnetisch markierten Zielspezies in ein Fluid über dem Chip;
Durchleiten eines Stroms durch den stromführenden Leiter, um die Gleichstromphase des elektromagnetischen Felds zu erzeugen, um die magnetisch markierten Biomoleküle anzuziehen; und
Erzeugen der zweiten Phase, eines alternierenden Magnetfelds, um die magnetisch markierten Moleküle von einer Leitung zu einer anderen über den Sensor hinweg steuerbar vor und zurück zu bewegen, wobei die erste und die zweite Phase gleichzeitig angelegt werden.

**Revendications**

**1.** Dispositif bioélectronique intégré dans une puce comprenant :

un conducteur porteur de courant ;
un capteur biomoléculaire situé à proximité du conducteur, dans lequel le conducteur est dans un motif ayant une pluralité de lignes à proximité étroite et le capteur est situé entre les lignes ; et
un électroaimant permettant une commande d'interaction biomoléculaire à modulation de fréquence via l'application de champs magnétiques variables à des biomolécules marquées magnétiquement,

dans lequel le dispositif est configuré pour générer des champs électromagnétiques en deux phases, une première phase de courant continu pour attirer les biomolécules marquées magnétiquement, et une seconde phase avec un champ magnétique alternatif et une modulation de fréquence pour déplacer de façon commandable les molécules marquées magnétiquement en va-et-vient d'une ligne à une autre à travers le capteur, dans lequel les première et seconde phases sont appliquées simultanément.

ment.

2.  Dispositif bioélectronique selon la revendication 1, dans lequel le capteur est un capteur magnétorésistif.

3.  Dispositif bioélectronique selon la revendication 1 ou 2, dans lequel le capteur et le conducteur forment ensemble une microunité électromagnétique agencée sur la puce.

4.  Dispositif bioélectronique selon la revendication 3, dans lequel une pluralité desdites unités sont présentes sur la puce, et elles sont adressables électroniquement.

5.  Dispositif bioélectronique selon l'une quelconque des revendications précédentes, dans lequel la surface de puce est fonctionnalisée avec une sonde.

6.  Dispositif bioélectronique selon la revendication 5, dans lequel la sonde est à motifs.

7.  Dispositif bioélectronique selon l'une quelconque des revendications précédentes, dans lequel le champ magnétique alternatif est dans le plan de la puce et orthogonal à la direction du conducteur proximal.

8.  Procédé d'utilisation du dispositif bioélectronique de l'une quelconque des revendications précédentes, comprenant :

    l'introduction d'espèces cibles marquées magnétiquement dans un fluide par-dessus la puce ;
    le passage d'un courant à travers le conducteur porteur de courant pour générer le champ électromagnétique de phase à courant continu pour attirer les biomolécules marquées magnétiquement ; et
    la génération du champ magnétique alternatif de seconde phase pour déplacer de façon commandable les molécules marquées magnétiquement en va-et-vient d'une ligne à une autre à travers le capteur, dans lequel les première et seconde phases sont appliquées simultané-

T = 3000Å

W = 10μm

S = 10μm

I = 40mA

L = 100μm

Fig. 1

Current line

sensor

sensor
contacts

Fig. 2

Fig. 3

Fig. 4

Fig. 5

Fig. 6

Fig. 7

Fig. 8

Fig. 9

(a)

(b)

Fig. 10

(a)

(b)

8mm

Fig. 11

Fig. 12

Fig. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 5981297 A

**Non-patent literature cited in the description**

• **LAGAE et al.** On-chip manipulation and magnetisation assessment of magnetic bead ensembles by integrated spin-valve sensors. *Journal of Applied Physics,* 2002, vol. 91 (10